(19) Europäisches Patentamt
European Patent Office
Office européen des brevets

(11) Veröffentlichungsnummer: **0 190 101 B1**

(12) **EUROPÄISCHE PATENTSCHRIFT**

(45) Veröffentlichungstag der Patentschrift: **22.07.92**

(51) Int. Cl.[5]: **C07C 209/18**, C07C 217/76, C07D 307/14, C07D 307/52

(21) Anmeldenummer: **86810038.9**

(22) Anmeldetag: **23.01.86**

(54) **Verfahren zur Herstellung von N-Alkylanilinen.**

(30) Priorität: **29.01.85 US 696235**

(43) Veröffentlichungstag der Anmeldung:
**06.08.86 Patentblatt 86/32**

(45) Bekanntmachung des Hinweises auf die Patenterteilung:
**22.07.92 Patentblatt 92/30**

(84) Benannte Vertragsstaaten:
**CH DE FR GB IT LI**

(56) Entgegenhaltungen:
**EP-A- 0 004 958**
**EP-A- 0 006 590**
**EP-A- 0 053 819**
**US-A- 3 878 131**

**HOUBEN-WEYL: "Methoden der organischen Chemie", Band XI/1: "Stickstoffverbindungen II", 1957, Seiten 126-134, Georg Thieme Verlag, Stuttgart, DE**

(73) Patentinhaber: **CIBA-GEIGY AG**
**Klybeckstrasse 141**
**CH-4002 Basel(CH)**

(72) Erfinder: **Rusek, Milos**
**Tiefengrabenstrasse 49**
**CH-4102 Binningen(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein Verfahren zur Herstellung von N-Alkylanilinen der Formel I

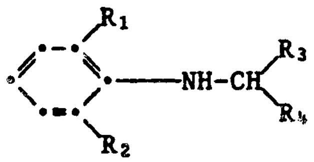

(I),

in welcher

| | |
|---|---|
| $R_1$ und $R_2$ | unabhängig voneinander $C_{1-6}$-Alkyl, $C_{1-6}$-Alkoxy oder Wasserstoff, |
| $R_3$ | $C_{1-6}$-Alkyl, $C_{3-6}$-Cycloalkyl, $C_{1-4}$-Alkoxy, $C_{1-4}$-Alkoxy-$C_{1-4}$-alkyl, Phenyl oder Wasserstoff, |
| $R_4$ | $C_{1-6}$-Alkyl, $C_{1-4}$-Alkoxy, Furfuryl, Tetrahydrofurfuryl oder Wasserstoff bedeuten, wobei $R_3$ und $R_4$ mit dem sie verbindenden Kohlenstoffatom einen gegebenenfalls ein- oder zweifach durch $C_{1-4}$-Alkyl substituierten 4-7-gliedrigen isocyclischen Ring oder einen Pyran- oder Tetrahydropyranring bilden können und einen Katalysator zur Durchführung dieses Verfahrens. |

Die N-Alkylaniline der Formel I sind wertvolle Zwischenprodukte zur Herstellung pestizider Wirkstoffe. Sie können mit Halogenacetylhalogeniden, wie Chloracetylchlorid oder Bromacetylbromid in die entsprechenden Acetanilide mit pestizider Wirkung übergeführt werden. Solche Acetanilide, ihre Herstellung und Verwendung sind beispielsweise in den US-Patentschriften 2,863,752, 3,345,151, 3,268,584, 3,952,056 und 3,937,730, in der französischen Patentschrift 1,339,001 sowie in der deutschen Patentschrift 2,305,495 und in der deutschen Auslegeschrift 2,328,340 beschrieben.

In der US-A-3 878 131 wird ein Dehydrierungs verfahren von Kohlenwasserstoffen unter Verwendung eines Katalysators beschrieben, der aus einem gemisch der Elemente Platin, Zinn, germanium umd einem Alkalimetall oder Erdalkalimetall bestehen, wobei Platin in Verhältnis zu den anderen Bestandteilen im Unterschuß enthalten ist.

Es ist bekannt, N-Alkylaniline durch Umsetzung von Anilinen mit Alkylhalogeniden, -tosylaten oder -phosphaten herzustellen. Bei Anwendung dieser Methode werden jedoch ausser den gewünschten N-Monoalkylanilinen stets beträchtliche Mengen N,N-Dialkylaniline gebildet. Dieses Verfahren ist daher wegen seiner zu geringen Selektivität für eine technische Herstellung von Verbindungen der Formel I ungeeignet. Ausserdem wirft dieses Verfahren ökologische Probleme auf, da die Abwässer stets grosse Mengen von Halogenwasserstoff, Toluolsulfonsäure oder Phosphorsäure bzw. von Salzen dieser Säuren enthalten.

Es wurde ferner bereits vorgeschlagen, Aniline in Gegenwart von Katalysatoren mit Alkoholen zu N-Alkylanilinen umzusetzen (vgl. Kirk-Othmer, Encyclopedia of Chemical Technolgy, 2nd Edition, Vol. 2, 412-13). Dabei wurden als Katalysatoren neben Aluminiumoxid, Aluminiumsilikat, einem Gemisch aus Phosphorsäure und Bentonit, Mineralsäuren, wie Chlorwasserstoff oder Schwefelsäure, auch wasserstoffübertragende Katalysatoren, verwendet. Beispielsweise wird nach einem im US-Patent 2,580,284 beschriebenen Verfahren Anilin in Gegenwart eines kupferhaltigen Tonerdekatalysators und in Gegenwart von Wasserstoff mit Methanol in einer Ausbeute von 96% der Theorie zu N-Methylanilin umgesetzt. Ferner kann Aethanol in Gegenwart von Raney-Nickel in einer Ausbeute von 80 bis 83% der Theorie zu N-Aethylanilin umgesetzt werden (vgl. J. Org. Chem. 21, 474- (1956) und J. Amer Chem. Soc. 77, 4052-54 (1955)). Bei der Umsetzung von Anilin und Methanol in Gegenwart von Kupferchromit und Wasserstoff wird in einheitlicher Reaktion N-Methylanilin in praktisch quantitativer Ausbeute gebildet (vgl. japanische Offenlegungsschrift 73 49,727; C.A. 79, (1973), 136.769w und deutsche Offenlegungsschrift 2.061.709.

Wie diese Ausführungen zeigen, führt die Umsetzung von Anilinen, welche an der ortho-Stelle des Benzolringes unsubstituiert sind, mit Alkoholen in Gegenwart von Wasserstoff und wasserstoffübertragenden Katalysatoren in ausgezeichneten Ausbeuten zu N-Alkylanilinen. Nach diesen Verfahren ist es jedoch nicht möglich o-mono- oder o,o-disubstituierte Aniline mit Alkoholen und insbesondere mit Alkoxyalkanolen mit guter Ausbeute zu den entsprechenden N-Alkylanilinen umsetzen.

Die N-Monoalkylierung von 2,6-Dialkylanilinen mit Alkoholen kann in Gegenwart eines kupferhaltigen Katalysators, der wenig Palladium oder Platin enthält, bei 200-350° nach US-P 4,183,868 durchgeführt werden. Während diese Alkylierung mit primären Alkoholen mit mässig bis guter Ausbeute verläuft, sind Umsatz, Selektivität und Ausbeute mit sekundären Alkoholen nicht zufriedenstellend.

Der vorliegenden Erfindung liegt daher die Aufgabe zugrunde, ein Verfahren bereitzustellen, nach dem

Aniline, sowie ihre o-mono- oder o,o-disubstituierten Derivate in guten Ausbeuten mit primären und sekundären Alkoholen zu den entsprechenden N-Alkylanilinen umgesetzt werden können.

Es wurde nun gefunden, das man N-Alkylaniline der Formel I

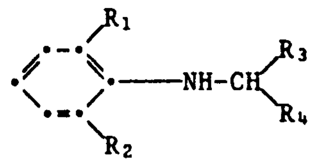

(I),

in welcher $R_1$ und $R_2$ die unter Formel I angegebenen Bedeutungen haben, durch Umsetzung eines Anilins der Formel II

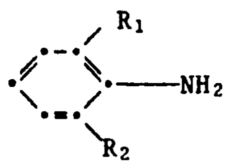

(II),

in welcher $R_1$ und $R_2$ die unter Formel I angegebene Bedeutung haben, mit einem Alkohol der Formel III

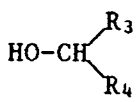

(III),

worin $R_3$ und $R_4$ die unter Formel I angegebene Bedeutung haben, in Gegenwart von Wasserstoff und eines Katalysators, in ausgezeichneter Ausbeute herstellen kann, wenn man die Umsetzung bei einer Temperatur von 150-300 °C und einem Druck von 1-6 bar in Gegenwart eines aus Kieselgel als Träger und 0,2-10 % Platin, 0,05-3 % einer Verbindung eines Elements der Gruppen Ia und/oder IIa des periodischen Systems bestehenden Katalysators durchführt, der zusätzlich mindestens eine Verbindung eines Elements der Gruppen Ib, IVa, IVb, Vb, VIIb und VIII in solcher Menge enthält, dass das Atomverhältnis von Platin zur Summe dieser Elemente 1:1 bis 6:1 beträgt.

Die vorliegende Erfindung betrifft vor allem ein Verfahren zur Herstellung von N-Alkylanilinen der Formel I, in welcher $R_1$ $C_{1-3}$-Alkyl oder Wasserstoff, $R_2$ $C_{1-3}$-Alkyl oder $C_{1-2}$-Alkoxy, $R_3$ $C_{1-3}$-Alkoxymethyl, $R_4$ Methyl oder Wasserstoff bedeuten und in welchen $R_3$ und $R_4$ einen mit dem sie verbindenden Kohlenstoffatom einen Cycloalkyl-, einen 2-Methylcyclohexyl- oder einen 2,6-Dimethylcyclohexylrest bilden.

Die vorliegende Erfindung betrifft insbesondere ein Verfahren zur Herstellung von N-Alkylanilinen der Formel I, worin $R_4$ $C_1$-$C_6$-Alkyl $C_1$-$C_4$-Alkoxy, Furfuryl oder Tetrahydrofurfuryl ist.

Die vorliegende Erfindung betrifft insbesondere auch Verfahren zur Herstellung von N-Alkylanilinen der Formel I, in welcher $R_3$ und $R_4$ mit dem sie verbindenden Kohlenstoffatom einen Pyran- oder Tetrahydropyranring bilden.

Die vorliegende Erfindung betrifft schliesslich insbesondere Verfahren zur Herstellung des 2-Methyl-6-äthyl-N-(1-methyl-2-methoxy-äthyl)-anilins und des 2,6-Diäthyl-N-2-propoxyäthylanilins.

Der bevorzugte Temperaturbereich für das erfindungsgemässe Verfahren liegt in der Regel bei 175-225 °, der bevorzugte Druckbereich bei 1-4 bar.

Der stündliche Durchsatz an Anilin der Formel II beträgt zweckmässig 0,5-10, vorzugsweise 1-5 Mol pro Liter Katalysator.

Das Molverhältnis des umzusetzenden Alkohols der Formel III zum Anilin der Formel II beträgt in der Regel 0,1:1 bis 10:1, vorzugsweise 1,1:1 bis 2,1:1.

Der Wasserstoffzusatz beträgt zweckmässig 0,5:1 bis 10:1, vorzugsweise 0,8:1 bis 1,2:1 Mol pro Mol eingesetztes Anilin der Formel II.

Als Träger für die erfindungsgemässen Katalysatoren kommen Kieselgele mit folgenden Eigenschaften in Betracht:

| | |
|---|---|
| Spezifische Oberfläche | 300-700 $m^2/g$ |
| Porenvolumen | 0,25-1,0 ml/g |
| Mittlerer Porenradius | 1 - 8 nm |
| Schüttgewicht | 0,25-0,9 g/ml |
| Gehalt an Wasser | 1 - 5 % |
| Eisen-, Aluminium- und Titangehalt total | max. 750 ppm |

Als Kieselgele können handelsübliche Produkte verwendet werden, sofern sie dieser Spezifikation entsprechen. Ferner können handelsübliche Produkte mit einem zu hohen Gehalt an Eisen, Aluminium und Titan durch Auswaschen mit verdünnten Säuren, wie beispielsweise 10 %ige wässrige Salzsäure, in erfindungsgemäss geeignete Träger überführt werden. In den hierbei resultierenden Produkten sollte die Summe von Eisen-, Aluminium- und Titangehalt unter 750 ppm, vorzugsweise unter 500 ppm liegen, wobei der Gehalt eines der genannten Metalle vorzugsweise unter 200 ppm liegen sollte.

Als besonders geeignet hat sich ein Kieselgel mit einer

| | |
|---|---|
| spezifischen Oberfläche von | 400-600 $m^2/g$ |
| Porenvolumen | 0,35-0,75 ml/g |
| Mittlerer Porenradius | 2 - 4 nm |
| Schüttgewicht: | 0,65-0,75 g/ml |
| Gehalt an Wasser | 1,5-2,5 % und |
| Gehalt an Eisen, Aluminium und Titan max. | 500 ppm |

erwiesen.

Der als Träger eingesetzte Kieselgel wird nach einem allfälligen Waschen mit verdünnter Säure durch Halten bei einer Temperatur von über 500 °C während mehreren Stunden stabilisiert.

Zur Herstellung des Katalysators werden eine geeignete Platinverbindung, wie z.B. Hexachlorplatinsäure und mindestens eine wasserlösliche Verbindung der Elemente der Gruppen Ib, IVa, IVb, Vb, VIIb und VIII des Periodischen Systems als Promotor in gelöster Form auf den vorher kalzinierten Silicagel als Träger so aufgetragen, dass die Lösung durch den Träger aufgesaugt wird (Tränken). Als Lösungsmittel eignet sich in den meisten Fällen Wasser.

In Wasser unlösliche Produkte, wie z.B. Germaniumtetrachlorid, werden in einem inerten organischen Lösungsmittel, wie z.B. Methylenchlorid, aufgelöst. Nach Auftragung dieser Produkte wird die resultierende Mischung in Vakuum bei 80 bis 150 °C getrocknet. Darauf wird mindestens eine Verbindung der Elemente der Gruppen Ia und IIa des Periodischen Systems als zweiter Promotor in gleicher Weise aufgetragen. Als Verbindungen eignen sich z.B. die Hydroxide oder Chloride. Die Auftragung der beiden Promotoren-Typen kann auch in umgekehrter Reihenfolge erfolgen.

Der anschliessend erneut getrocknete Katalysator wird durch Halten bei Temperaturen um 400 °C während mehreren Stunden kalziniert. Der so erhältliche rohe Katalysator wird nach Abkühlen in den Reaktor eingesetzt und zum Anfang eines Versuchs aktiviert.

Der erfindungsgemässe Katalysator ist neu und ebenfalls Gegenstand der vorliegenden Erfindung.

Die Aktivierung des rohen Katalysators erfolgt im Reaktor in einem Gasstrom unter atmosphärischem Druck während mehreren Stunden bei 140 °C, wobei die Zusammensetzung des Gases von Stickstoff allmählich auf Wasserstoff geändert wird. Der schliesslich entstandene Katalysator besteht aus Kieselgel, Platin und aus mindestens einer Verbindung der Elemente der Gruppen Ib, IVa, IVb, Vb, VIIb und VIII und aus mindestens einer Verbindung der Elemente der Gruppen Ia und IIa des Periodischen Systems und kann für das erfindungsgemässe Verfahren eingesetzt werden.

Von den neuen Katalysatoren, in deren Gegenwart das erfindungsgemässe Verfahren zur Herstellung der Verbindungen der Formel I durchgeführt wird, sind folgende Zusammensetzungen bevorzugt:

a)
- 0,5-6 % Platin,
- 0,1-2 % mindestens einer Verbindung der Elemente Lithium, Natrium, Kalium, Rubidium, Cesium, Beryllium, Magnesium, Calcium, Strontium oder Barium
- mindestens eine Verbindung der Elemente Kupfer, Germanium, Zinn, Blei, Titan, Niob, Mangan und Ruthen, wobei das Atomverhältnis von Platin zur Summe dieser Elemente 2,5:1 bis 3,5:1 beträgt,

b)

- 0,5-6 % Platin
- 0,1-2 % mindestens einer Verbindung der Elemente Lithium, Natrium, Kalium, Rubidium, Cesium, Beryllium, Magnesium, Calcium, Strontium oder Barium
- eine Rhenium-Verbindung, wobei das Atomverhältnis von Platin zu Rhenium 0,5:1 bis 1,5:1 beträgt

Von den neuen Katalysatoren wird folgende Zusammensetzung besonders bevorzugt:

- 3-5 % Platin
- 0,1-2 % einer Calcium-Verbindung
- eine Zinn-II-Verbindung im Atomverhältnis Platin:Zinn = 2,5-3,5.

Ein nach langdauerndem Betrieb desaktivierter Katalysator kann nach einer durch Halten bei 450-600° im Luftstrom erfolgten mehrstündigen Regeneration analog zur bereits beschriebenen Aktivierung reaktiviert und wieder eingesetzt werden.

Das erfindungsgemässe Verfahren wird zweckmässig kontinuierlich in Festbettreaktoren oder Rieselapparaten durchgeführt. Das durch den Reaktor strömende Gemisch kann entweder rein gasförmig sein oder eine Gas-und Flüssigphase bilden.

Die Einführung der beiden Edukte in den Reaktor kann entweder voneinander getrennt oder als eine im erwünschten Verhältnis vorbereitete Mischung erfolgen. Es ist vorteilhaft, die Edukte im dünnflüssigen Zustand zu dosieren, wozu sie gegebenenfalls erwärmt werden müssen. Vor ihrem Eintritt in den Reaktor sollen die Edukte gegebenenfalls noch weiter erhitzt und auch verdampft werden.

Das resultierende Reaktionsgemisch kann gaschromatographisch analysiert werden. Seine präparative Auftrennung kann mit Hilfe der üblichen Trennmethoden erfolgen. Der Wasserstoff und die nicht umgesetzten Edukte können bei der technischen Durchführung des Verfahrens erneut eingesetzt werden.

Durch das erfindungsgemässe Verfahren können Anilin und seine o-mono- und o,o-disubstituierten Derivate mit Alkoholen mit sehr guter Ausbeute alkyliert werden. Mit Hilfe des erfindungsgemässen Verfahrens wird die Alkylierung darüber hinaus auch mit sekundären Alkoholen mit gleich guter Ausbeute ermöglicht. Dadurch können die Zwischenprodukte der Formel I, ausgehend von billigen, leicht zugänglichen Alkoholen, mit hohem Umsatz und ausgezeichneter Selektivität hergestellt werden. Das Verfahren kann aufgrund der kurzen Reaktionszeiten leicht kontinuierlich durchgeführt werden und ist daher für eine technische Herstellung von Zwischenprodukten der Formel I gut geeignet. Die Aktivität des Katalysators nimmt erst nach etwa einem Tag Dauerbetrieb merkbar ab. Ein Dauerbetrieb des Reaktors kann durch cyclische Wiederholung der Arbeitsphasen, Aktivierung, Reaktion, Regeneration, dann Reaktivierung, Reaktion usw. aufrechterhalten werden. Bei der Durchführung der Reaktion im technischen Masstab können zwei oder mehrere Reaktoren parallel zueinander mit entsprechend verschobenen Arbeitsphasen eine gleichmässige Dauerproduktion gewährleisten.

Das Verfahren bietet ferner gegenüber denjenigen Verfahren, bei denen die Alkylierung mit Alkylhalogeniden oder -tosylaten durchgeführt wird, in ökologischer Hinsicht wesentliche Vorteile, da die bei diesen Verfahren auftretende hohe Salzbelastung des Abwassers wegfällt.

Das erfindungsgemässe Verfahren wird durch die nachfolgenden Beispiele näher erläutert.

<u>Beispiel 1</u>: <u>Herstellung von 2-Methyl-6-äthyl-N-(1-methyl-2-methoxyäthyl)-anilin</u>

9,5 g eines mittelporigen Kieselgels mit folgenden charakteristischen Kenndaten:

| | | |
|---|---|---|
| Oberfläche (BET) | | 468 $m^2/g$ |
| Porenvolumen | | 0,62 ml/g |
| Mittlerer Porenradius | | 2,65 nm |
| Gehalt an | Calcium | <10 ppm |
| | Magnesium | <10 ppm |
| | Eisen | 115 ppm |
| | Kalium | <20 ppm |
| | Natrium | <50 ppm |
| | Aluminium | 155 ppm |
| | Titan | 145 ppm |
| | Wasser | 1,8 % |

werden mit 7,5 ml einer wässrige Lösung, bestehend aus 2,5 g Hexachlorplatinsäure.x$H_2O$ (5,1 mMol) und 0,38 g Zinn-II-chlorid.2$H_2O$ (1,7 mMol) getränkt, gut vermischt und 2 Stunden lang bei 90°C im Vakuum

getrocknet. Das resultierende Gemisch wird mit 6 ml einer wässrigen Lösung bestehend aus 0,37 g Calciumchlorid-Dihydrat (2,5 mMol) zusätzlich getränkt und vermischt. Nach erneutem Trocknen bei 90 °C im Vakuum wird das Gemisch durch fünfstündiges Halten bei 350 °C kalziniert, wodurch 15 ml zu aktivierender Katalysator gewonnen werden.

4,6 ml dieses rohen Katalysators werden in einem Mikroreaktor mit einem 50 ml/Min starken Wasserstoff/Stickstoff-Gasstrom bei 140 °C während 1 1/2 Stunden aktiviert, wobei der Wasserstoffgehalt während der ersten Stunde linear von 0 auf 100% erhöht wird. Anschliessend wird ein Wasserstoffstrom von 3,75 ml/Min (9,2 mMol/Std.) eingestellt und die Temperatur im Reaktor auf 200 ° gestellt. Hierzu werden 3 ml/Std einer Mischung von 135,2 g (1 Mol) 2-Methyl-6-äthylanilin und 180,2 g (2 Mol) 1-Methoxy-propanol-2 in den Reaktor gepumpt, verdampft und gasförmig über die Katalysatorschicht geführt, was einer Dosierung von 9,2 mMol/Std 2-Methyl-6-äthylanilin und einem Durchsatz von 2 Mol 2-Methyl-6-äthylanilin pro Stunde und Liter Katalysator entspricht. Während 15 Stunden Reaktionszeit werden folgende Resultate erzielt.

| | | | |
|---|---|---|---|
| Gesamtumsatz bezogen auf 2-Methyl-6-äthylanilin | | $(U_A)$: | 65,4 % |
| Selektivität " | " | $(S_A)$: | 93,7 % |

Anschliessend wird die Wasserstoff- und Eduktzugabe durch einen 50 ml/Min starken Stickstoff/Luftstrom ersetzt, wobei während 1 1/2 Stunden der Luftanteil linear von 0 auf 100% und die Temperatur von 190 auf 450 °C erhöht wird. Nach 3stündigem Halten des Katalysators unter diesen Bedingungen wird er im Stickstoffstrom auf 140 °C abgekühlt und wie oben bereits beschrieben, mit Wasserstoff aktiviert.

Anschliessend wird mit dem bereits beschriebenen Cyclus begonnen, der total 24 Stunden lang dauert. Die Katalysator-Aktivität steigt nach den ersten Cyclen gegenüber dem ersten Durchgang an.

Die Resultate zeigen erst nach mehrwöchigem Betrieb ein merkbares Absinken der Selektivität.

Nach dem erfindungsgemässen Verfahren werden mit demselben Katalysator weitere Beispiele mit den folgenden Resulaten durchgeführt. (Siehe Tabelle).

Tabelle

| Nr. | Edukte und Produkte der Formeln I + II $R_1$ | $R_2$ | $R_4$ | $R_3$ | Durchsatz Mol Anilin (II) pro L.Kat.Std. | Temp. °C | Umsatz $U_A$ * | Selektivität $S_A$ * |
|---|---|---|---|---|---|---|---|---|
| 1 | $CH_3$- | $C_2H_5$- | $-CH_2OCH_3$ | $CH_3$- | 2 | 200 | 65,4 | 93,7 |
| 2 | H- | $CH_3$- | $-CH_2OCH_3$ | H- | 6 | 190 | 75,5 | 94,2 |
| 3 | H- | $CH_3$- | $-CH_2-OCH_3$ | $CH_3$- | 4 | 200 | 94,3 | 98,7 |
| 4 | H- | $C_2H_5$- | $-CH_2-OCH_3$ | $CH_3$- | 4 | 200 | 92,1 | 98,6 |
| 5 | H- | $OCH_3$- | $-CH_2OCH_3$ | H- | 1 | 175 | 92,7 | 91,7 |
| 6 | H- | $OCH_3$- | $-CH_3OCH_3$ | $CH_3$- | 1 | 225 | 79,5 | 94,7 |
| 7 | H- | $OC_2H_5$- | $-CH_2OCH_3$ | $CH_3$- | 2 | 200 | 97,5 | 97,0 |
| 8 | $CH_3$- | $CH_3$- | $-CH_2OCH_3$ | H- | 4 | 190 | 38,1 | 94,9 |
| 9 | $CH_3$- | $CH_3$- | $-CH_2OCH_3$ | $CH_3$- | 4 | 200 | 56,2 | 95,6 |
| 10 | $C_2H_5$- | $C_2H_5$- | $-CH_2O-nC_3H_7$ | H- | 4 | 190 | 52,1 | 89,7 |
| 11 | $CH_3$- | $CH_3$- | $-CH_2O-nC_3H_7$ | H- | 4 | 190 | 52,1 | 89,7 |
| 12 | $CH_3$- | $C_2H_5$- | $-CH_2OCH_3$ | H- | 4 | 190 | 62,0 | 91,1 |
| 13 | $C_2H_5$- | $C_2H_5$- | $-CH_2OCH_3$ | $CH_3$- | 4 | 200 | 52,3 | 89,0 |

<u>Tabelle</u> (Fortsetzung)

| Nr. | Edukte und Produkte der Formeln I + II $R_1$ | $R_2$ | $R_4$ | $R_3$ | Durchsatz Mol Anilin (II) pro L.Kat.Std. | Temp. °C | Umsatz $U_A$ * | Selektivität $S_A$ * | |
|---|---|---|---|---|---|---|---|---|---|
| 14 | i-$C_3H_5$- | i-$C_3H_7$- | -$CH_2OCH_3$ | $CH_3$- | 2 | 200 | 36,2 | 79,1 | |
| 15 | i-$C_3H_7$- | i-$C_3H_7$- | -$CH_2OCH_3$ | $CH_3$- | 2 | 200 | 53,7 | 86,0 | *** |
| 16 | $CH_3$- | $CH_3$- | -$CH_2$-$(CH_2)_3$-$CH_2$- | | 2 | 200 | 71,7 | 91,2 | ** |
| 17 | $CH_3$- | H- | -CH(2-$CH_3$)$(CH_2)_3$-$CH_2$- | | 2 | 200 | 65,4 | 95,4 | |
| 18 | $CH_3$- | H- | -CH(2-$CH_3$)-($CH_2$)-CH(6-$CH_3$)- | | 2 | 200 | 60,9 | 94,0 | |
| 19 | H- | H- | -$CH_2OCH_3$ | H- | 6 | 190 | 51,1 | 95,8 | |
| 20 | H- | H- | -$CH_2OCH_3$ | $CH_3$- | 8 | 225 | 66,9 | 97,8 | |
| 21 | $CH_3$- | $CH_3$- | 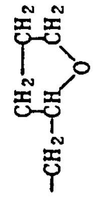 | -H | 2 | 190 | 68,3 | 90,2 | **** |
| 22 | $CH_3$- | $CH_3$- | 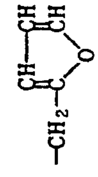 | -H | 2 | 200 | 67,5 | 91,3 | ***** |
| 23 | $CH_3$- | $CH_3$- | -$(CH_2)_2$-O-$(CH_2)_2$- | | 2 | 200 | 65,7 | 89,2 | **** |
| 24 | $CH_3$- | H- | -CH(3-$CH_3$)-$CH_2$-O-$(CH_2)_2$- | | 2 | 200 | 60,4 | 91,2 | **** |

*) s. Beispiel 1
**) $R_3$ und $R_4$ zusammen, gemäss Definition (Seite 1)
***) Bei der Herstellung des Katalysators gemäss Beispiel 1 wurde das Calciumchlorid durch 2,5 Mmol Bariumchlorid ersetzt.
****) Summe der erwünschten und der dehydrierten Produkte
*****) Summe der erwünschten und der hydrierten Produkte.

## Patentansprüche

**1.** Verfahren zur Herstellung von N-Alkylanilinen der Formel I

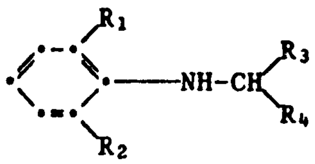

(I),

in welcher

$R_1$ und $R_2$ unabhängig voneinander $C_{1-6}$-Alkyl, $C_{1-6}$-Alkoxy oder Wasserstoff,

$R_3$ $C_{1-6}$-Alkyl, $C_{3-6}$-Cycloalkyl, $C_{1-4}$-Alkoxy, $C_{1-4}$-Alkoxy-$C_{1-4}$-alkyl, Phenyl oder Wasserstoff,

$R_4$ $C_{1-6}$-Alkyl, $C_{1-4}$-Alkoxy, Furfuryl, Tetrahydrofurfuryl oder Wasserstoff bedeuten, wobei $R_3$ und $R_4$ mit dem sie verbindenen Kohlenstoffatom einen gegebenenfalls ein- oder zweifach durch $C_{1-4}$-Alkyl substituierten 4-7-gliedrigen isocyclischen Ring oder einen Pyran- oder Tetrahydropyranring bilden können, durch Umsetzung eines Anilins der Formel II

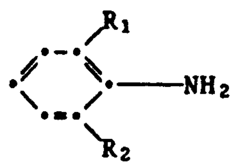

(II),

in welcher $R_1$ und $R_2$ die unter Formel I angegebene Bedeutung haben, mit einem Alkohol der Formel III

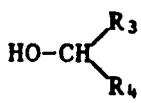

(III),

worin $R_3$ und $R_4$ die unter Formel I angegebene Bedeutung haben, in Gegenwart von Wasserstoff und eines Katalysators, dadurch gekennzeichnet, dass man die Umsetzung bei einer Temperatur von 150-300 °C und einem Druck von 1-6 bar in Gegenwart eines aus Kieselgel als Träger und 0,2-10 % Platin, 0,05-3 % einer Verbindung eines Elements der Gruppen Ia und/oder IIa des periodischen Systems bestehenden Katalysators durchführt, der zusätzlich mindestens eine Verbindung eines Elements der Gruppen Ib, IVa, IVb, Vb, VIIb und VIII in solcher Menge enthält, dass das Atomverhältnis von Platin zur Summe dieser Elemente 1:1 bis 6:1 beträgt.

2. Verfahren nach Anspruch 1, dadurch gekennzeichnet, dass man die Umsetzung zwischen 175-225 °C durchführt.

3. Verfahren nach Anspruch 1, dadurch gekennzeichnet, dass man die Reaktion bei 1-4 bar durchführt.

4. Verfahren nach Anspruch 1, dadurch gekennzeichnet, dass die Umsetzung mit einem stündlichen Durchsatz von 0,5-10 Mol Anilin der Formel II pro Liter Katalysator durchgeführt wird.

5. Verfahren nach Anspruch 4, dadurch gekennzeichnet, dass die Umsetzung mit einem stündlichen Durchsatz von 1-5 Mol Anilin der Formel II pro Liter Katalysator durchgeführt wird.

6. Verfahren nach Anspruch 1, dadurch gekennzeichnet, dass das Molverhältnis des Alkohols der Formel III zum Anilin der Formel II 0,1:1 bis 10:1 beträgt.

7. Verfahren nach Anspruch 6, dadurch gekennzeichnet, dass das Molverhältnis des Alkohols der Formel III zum Anilin der Formel II 1,1:1 bis 2,1:1 beträgt.

8. Verfahren nach Anspruch 1, dadurch gekennzeichnet, das der Zusatz von Wasserstoff im Molverhältnis

0,5:1 bis 10:1 pro Mol Anilin der Formel II erfolgt.

**9.** Verfahren nach Anspruch 8, dadurch gekennzeichnet, dass der Zusatz von Wasserstoff im Molverhältnis 0,8:1 bis 1,2:1 pro Mol Anilin der Formel II erfolgt.

**10.** Verfahren nach Anspruch 1, dadurch gekennzeichnet, dass der Katalysator aus 0,5-6 % Platin, aus 0,1-2 % mindestens einer der Verbindungen der Elemente Lithium, Natrium, Kalium, Rubidium, Cesium, Beryllium, Magnesium, Calcium, Strontium oder Barium und aus mindestens einer der Verbindungen der Elemente Kupfer, Germanium, Zinn, Blei, Titan, Niob, Rhenium, Mangan und Ruthen besteht, wobei das Atomverhältnis von Platin zur Summe dieser Elemente 2,5:1 bis 3,5:1 beträgt.

**11.** Verfahren nach Anspruch 1, dadurch gekennzeichnet, dass der Katalysator aus 0,5-6 % Platin, aus 0,1-2 % mindestens einer der Verbindungen der Elemente Lithium, Natrium, Kalium, Rubidium, Cesium, Beryllium, Magnesium, Calcium, Strontium oder Barium und aus einer Rheniumverbindung besteht, wobei das Atomverhältnis Platin zum Rhenium 0,5:1 bis 1,5:1 beträgt.

**12.** Verfahren nach Anspruch 1, dadurch gekennzeichnet, dass $R_1$ $C_{1-3}$-Alkyl oder Wasserstoff, $R_2$ $C_{1-3}$-Alkyl oder $C_{1-2}$-Alkoxy, R3 $C_{1-3}$-Alkoxymethyl, $R_4$ Methyl oder Wasserstoff bedeuten und in welchen $R_3$ und $R_4$ einen mit dem sie verbindenden Kohlenstoffatom einen Cyclohexyl-, einen 2-Methylcyclohexyl- oder einen 2,6-Dimethylcyclohexylrest bilden.

**13.** Verfahren nach Anspruch 1, dadurch gekennzeichnet, dass $R_4$ $C_1$-$C_6$-Alkyl, $C_1$-$C_4$-Alkoxy, Furfuryl oder Tetrahydrofurfuryl bedeutet.

**14.** Verfahren nach Anspruch 1, dadurch gekennzeichnet, dass $R_3$ und $R_4$ mit dem sie verbindenden Kohlenstoffatom einen Pyran- oder Tetrahydropyranring bilden.

**15.** Verfahren nach Anspruch 1, dadurch gekennzeichnet, dass man 2-Methyl-6-äthyl-anilin mit 1-Methoxypropanol-2 im Molverhältnis 1:1-2,1 unter Zusatz von 1 Mol Wasserstoff pro Mol 2-Methyl-6-äthyl-anilinin Gegenwart eines Katalysators, bestehend aus 3-5 % Pt, aus 0,1-2 % einer Calcium-Verbindung und aus einer Zinn-II-Verbindung im Atomverhältnis Platin-Zinn = 2,5-3,5 bei 200° und unter Normaldruck zum 2-Methyl-6-äthyl-N-(1-methyl-2-methoxy-äthyl)-anilin umsetzt.

**16.** Verfahren nach Anspruch 1, dadurch gekennzeichnet, dass man 2,6-Diäthylanilin mit 2-Propoxyäthanol im Molverhältnis 1:1-2,1 unter Zusatz von 1 Mol Wasserstoff pro Mol 2,6-Diäthylanilin in Gegenwart eines Katalysators, bestehend aus 3-5 % Pt, aus 0,1-2 % einer Calcium-Verbindung und aus einer Zinn-II-Verbindung im Atomverhältnis Platin-Zinn = 2,5-3,5 bei 190° und unter Normaldruck zum 2,6-Diäthyl-N-2-propoxyäthylanilin umsetzt.

**17.** Katalysator auf der Basis von Kieselgel mit einer spezifischen Oberflächevon 300 - 700$m^2$/g, einem Porenvolumen von 0,25 bis 1,0 ml/g, einem mittleren Porenradius von 1 - 8 nm,einem Schüttgewicht von 0,25 - 0,9 g/ml, einem Wassergehalt von 1 - 5 % und mit einem Totalgehalt von Eisen, Aluminium und Titan von weniger als 750 ppm als Träger, enthaltend

a) 0,5 bis 6% Platin,

b) 0,1 bis 2 % mindestens einer Verbindung eines Elements der Gruppen Ia und/oder IIa des periodischen Systems und

c) mindestens eine Verbindung eines Elements der Gruppen Ib, IVa, IVb, Vb, VIIb und VIII, wobei das Atomverhältnis von Platin zur Summe der Elemente aus der Gruppe b) und c) 1:1 bis 6:1 beträgt.

**18.** Katalysator nach Anspruch 17, dadurch gekennzeichnet, dass das Kieselgel eine spezifische Oberfläche von 400-600 $m^2$/g, ein Porenvolumen von 0,35-0,75 ml/g, einen mittleren Porenradius von 2-4 nm, und ein Schüttgewicht von 0,65-0,75 g/ml aufweist, 1,5-2,5 % Wasser enthält und dass der Gehalt an Eisen, Aluminium und Titan total 500 ppm nicht überschreitet.

**19.** Katalysator auf der Basis von Kieselgel als Träger enthaltend

a) 0,2 bis 10 % Platin,

b) 0,05 bis 3 % einer Verbindung eines Elements der Gruppen Ia und/oder IIa des periodischen

Systems und
c) mindestens eine Verbindung eines Elements der Gruppen Ib, IVa, IVb, Vb, VIIb und VIII, wobei das Atomverhältnis von Platin zur Summe der Elemente aus der Gruppe b) und c) 1:1 bis 6:1 beträgt.

**20.** Katalysator nach Anspruch 19, bestehend aus 0,5-6 % Platin, aus 0,1-2 % mindestens einer Verbindung der Elemente Lithium, Natrium, Kalium, Rubidium, Cesium, Beryllium, Magnesium, Calcium, Strontium oder Barium und aus mindestens einer Verbindung der Elemente Kupfer, Germanium, Zinn, Blei, Titan, Niob, Mangan und Ruthen, wobei das Atomverhältnis Von Platin zur Summe dieser Elemente 2,5:1 bis 3,5:1 beträgt.

**21.** Katalysator nach Anspruch 19, bestehend aus 0,5-6 % Platin, aus 0,1-2 % mindesten einer Verbindung der Elemente Lithium, Natrium, Kalium, Rubidium, Cesium, Beryllium, Magnesium, Calcium, Strontium oder Barium und aus einer Rheniumverbindung, wobei das Atomverhältnis von Platin zu Rhenium 0,5:1 bis 1,5:1 beträgt.

**22.** Katalysator nach Anspruch 19 bestehend aus 3-5% Platin, aus 0,1-2 % einer Calcium-Verbindung und aus einer Zinn-II-Verbindung im Atomverhältnis Platin:Zinn = 2,5:1 bis 3,5:1.

## Claims

**1.** A process for the preparation of an N-alkylaniline of the formula I

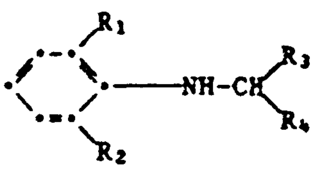

(I)

wherein $R_1$ and $R_2$ are each independently $C_1$-$C_6$alkyl, $C_1$-$C_6$alkoxy or hydrogen, $R_3$ is $C_1$-$C_6$alkyl, $C_3$-$C_6$cycloalkyl, $C_1$-$C_4$alkoxy, $C_1$-$C_4$alkoxy-$C_1$-$C_4$alkyl, phenyl or hydrogen, $R_4$ is $C_1$-$C_6$alkyl, $C_1$-$C_4$alkoxy, furfuryl, tetrahydrofurfuryl or hydrogen, and $R_3$ and $R_4$ together with the carbon atom linking them can form a 4- to 7-membered isocyclic ring which is unsubstituted or substituted by one or two $C_1$-$C_4$alkyl groups, or a pyran or tetrahydropyran ring, by reacting an aniline of the formula II

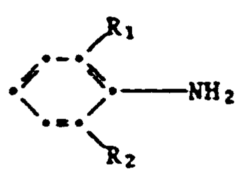

(II),

wherein $R_1$ and $R_2$ are as defined for formula I, with an alcohol of the formula III

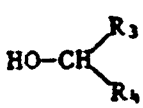

(III),

wherein $R_3$ and $R_4$ are as defined for formula I, in the presence of hydrogen and a catalyst, which process comprises carrying out the reaction in the temperature range from 150° to 300°C and under a pressure of 1 to 6 bar, in the presence of a catalyst consisting of silica gel as carrier and 0.2 to 10 % of platinum and 0.05 to 3 % of a compound of an element of groups Ia and/or IIa of the Periodic Table, said catalyst additionally comprising at least one compound of an element of groups Ib, IVa, IVb, Vb, VIIb and VIII in an amount such that the atomic ratio of platinum to the sum of those elements is 1:1 to 6:1.

2. A process according to claim 1, which comprises carrying out the reaction at from 175 to 225° C.

3. A process according to claim 1, which comprises carrying out the reaction in the temperature range from 1 to 4 bar.

4. A process according to claim 1, which comprises carrying out the reaction with an hourly rate of addition of 0.5 to 10 moles of aniline of the formula II per litre of catalyst.

5. A process according to claim 4, which comprises carrying out the reaction with an hourly rate of addition of 1 to 5 moles of aniline of the formula II per litre of catalyst.

6. A process according to claim 1, wherein the molar ratio of the alcohol of the formula III to the aniline of the formula II is 0.1:1 to 10:1.

7. A process according to claim 6, wherein the molar ratio of the alcohol of the formula III to the aniline of the formula II is 1.1:1 to 2.1:1.

8. A process according to claim 1, wherein the addition of hydrogen is effected in the molar ratio of 0.5:1 to 10:1 per mole of aniline of the formula II.

9. A process according to claim 8, wherein the addition of hydrogen is effected in the molar ratio of 0.8:1 to 1.2:1 per mole of aniline of the formula II.

10. A process according to claim 1, wherein the catalyst consists of 0.5 to 6 % of platinum, 0.1 to 2 % of at least one compound of the elements lithium, sodium, potassium, rubidium, caesium, beryllium, magnesium, calcium, strontium and barium, and at least one compound of the elements copper, germanium, tin, lead, titanium, niobium, rhenium, manganese and ruthenium, with the atomic ratio of platinum to the sum of those elements being 2.5:1 to 3.5:1.

11. A process according to claim 1, wherein the catalyst consists of 0.5 to 6 % of platinum, 0.1 to 2 % of at least one compound of the elements lithium, sodium, potassium, rubidium, caesium, beryllium, magnesium, calcium, strontium and barium, and a rhenium compound, with the atomic ratio of platinum to rhenium being 0.5:1 to 1.5:1.

12. A process according to claim 1, wherein $R_1$ is $C_1$-$C_3$alkyl or hydrogen, $R_2$ is $C_1$-$C_3$alkyl or $C_1$-$C_2$alkoxy, $R_3$ is $C_1$-$C_3$alkoxymethyl, $R_4$ is methyl or hydrogen, and wherein $R_3$ and $R_4$ together with the carbon atom linking them form a cyclohexyl, 2-methylcyclohexyl or 2,6-dimethylcyclohexyl radical.

13. A process according to claim 1, wherein $R_4$ is $C_1$-$C_6$alkyl, $C_1$-$C_4$alkoxy, furfuryl or tetrahydrofurfuryl.

14. A process according to claim 1, wherein $R_3$ and $R_4$ together with the carbon atom linking them form a pyran or tetrahydropyran ring.

15. A process according to claim 1, which comprises reacting 2-methyl-6-ethylaniline with 1-methoxypropan-2-ol in the molar ratio of 1:1 to 2.1 with the addition of 1 mole of hydrogen per mole of 2-methyl-6-ethylaniline, in the presence of a catalyst consisting of 3 to 5 % of platinum, 0.1 to 2 % of a calcium compound, and a tin(II) compound in the atomic ratio of platinum:tin = 2.5 to 3.5, at 200° and under normal pressure to give 2-methyl-6-ethyl-N-(1-methyl-2-methoxyethyl)aniline.

16. A process according to claim 1, which comprises reacting 2,6-diethylaniline with 2-propoxyethanol in the molar ratio of 1:1 to 2.1 with the addition of 1 mole of hydrogen per mole of 2,6-diethylaniline, in the presence of a catalyst consisting of 3 to 5 % of platinum, 0.1 to 2 % of a calcium compound, and a tin-(II) compound in the atomic ratio of platinum:tin = 2.5 to 3.5, at 190° and under normal pressure to give 2,6-diethyl-N-2-propoxyethylaniline.

17. A catalyst based on silica gel having a specific surface area of 300 to 700 $m^2/g$, a pore volume of 0.25 to 1.0 ml/g, an average pore radius of 1 to 8 nm, a bulk density of 0.25 to 0.9 g/ml, a water content of 1 to 5 % and a total content of iron, aluminium and titanium of less than 750 ppm, as carrier, comprising

a) 0.5 to 6 % of platinum,
b) 0.1 to 2 % of at least one compound of an element of groups Ia and/or IIa of the Periodic Table and
c) at least one compound of an element of groups Ib, IVa, IVb, Vb, VIIb and VIII, with the atomic ratio of platinum to the sum of the elements from groups b) and c) being 1:1 to 6:1.

**18.** A catalyst according to claim 17, wherein the silica gel has a specific surface area of 400 to 600 $m^2/g$, a pore volume of 0.35 to 0.75 ml/g, an average pore radius of 2 to 4 nm and a bulk density of 0.65 to 0.75 g/ml and contains 1.5 to 2.5 % water, and wherein the total content of iron, aluminium and titanium does not exceed 500 ppm.

**19.** A catalyst based on silica gel as carrier, comprising
a) 0.2 to 10 % of platinum,
b) 0.05 to 3 % of a compound of an element of groups Ia and/or IIa of the Periodic Table and
c) at least one compound of an element of groups Ib, IVa, IVb, Vb, VIIb and VIII, with the atomic ratio of platinum to the sum of the elements from groups b) and c) being 1:1 to 6:1.

**20.** A catalyst according to claim 19, consisting of 0.5 to 6 % of platinum, 0.1 to 2 % of at least one compound of the elements lithium, sodium, potassium, rubidium, caesium, beryllium, magnesium, calcium, strontium and barium, and at least one compound of the elements copper, germanium, tin, lead, titanium, niobium, manganese and ruthenium, with the atomic ratio of platinum to the sum of those elements being 2.5:1 to 3.5:1.

**21.** A catalyst according to claim 19, consisting of 0.5 to 6 % of platinum, 0.1 to 2 % of at least one compound of the elements lithium, sodium, potassium, rubidium, caesium, beryllium, magnesium, calcium, strontium and barium, and a rhenium compound, with the atomic ratio of platinum to rhenium being 0.5:1 to 1.5:1.

**22.** A catalyst according to claim 19, consisting of 3 to 5 % of platinum, 0.1 to 2 % of a calcium compound, and a tin(II) compound in the atomic ratio of platinum:tin = 2.5:1 to 3.5:1.

**Revendications**

**1.** Procédé de préparation des N-alkylanilines de formule I

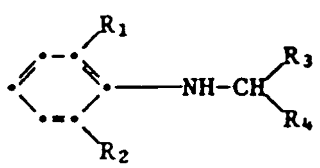

(I),

dans laquelle

$R_1$ et $R_2$ représentent chacun, indépendamment l'un de l'autre, un groupe alkyle en C1-C6, alcoxy en C1-C6 ou l'hydrogène,

$R_3$ représente un groupe alkyle en C1-C6, cycloalkyle en C3-C6, alcoxy en C1-C4, (alcoxy en C1-C4)-alkyle en C1-C4, phényle ou l'hydrogène,

$R_4$ représente un groupe alkyle en C1-C6, alcoxy en C1-C4, furfuryle, tétrahydrofurfuryle ou l'hydrogène, $R_3$ et $R_4$ pouvant également former ensemble et avec l'atome de carbone auquel ils sont reliés un noyau isocyclique de 4 à 7 chaînons portant éventuellement un ou deux substituants alkyle en C1-C4 ou un noyau pyranne ou tétrahydropyranne,

par réaction d'une aniline de formule II

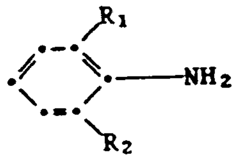

(II),

dans laquelle $R_1$ et $R_2$ ont les significations indiquées en référence à la formule I, avec un alcool de formule III

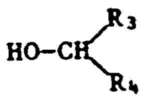

(III),

dans laquelle $R_3$ et $R_4$ ont les significations indiquées en référence à la formule I, en présence d'hydrogène et d'un catalyseur, caractérisé en ce que l'on effectue la réaction à une température de 150 à 300 ° C et une pression de 1 à 6 bar en présence d'un catalyseur consistant en gel de silice qui sert de support et 0,2 à 10% de platine, 0,05 à 3% d'un composé d'un élément des groupes Ia et/ou IIa de la Classification Périodique et qui contient en outre au moins un composé d'un élément des groupes Ib, IVa, IVb, Vb, VIIb et VIII en quantités correspondant a un rapport atomique de 1 : 1 à 6 : 1 entre le platine et la somme de ces éléments.

**2.** Procédé selon la revendication 1, caractérisé en ce que l'on effectue la réaction entre 175 et 225 ° C.

**3.** Procédé selon la revendication 1, caractérisé en ce que l'on effectue la réaction à une pression de 1 à 4 bar.

**4.** Procédé selon la revendication 1, caractérisé en ce que la réaction est effectuée avec un débit horaire de 0,5 à 10 mol de l'aniline de formule II par litre de catalyseur.

**5.** Procédé selon la revendication 4, caractérisé en ce que la réaction est effectuée avec un débit horaire de 1 à 5 mol de l'aniline de formule II par litre de catalyseur.

**6.** Procédé selon la revendication 1, caractérisé en ce que le rapport molaire entre l'alcool de formule III et l'aniline de formule II va de 0,1 : 1 à 10 : 1.

**7.** Procédé selon la revendication 6, caractérisé en ce que le rapport molaire entre l'alcool de formule III et l'aniline de formule II va de 1,1 : 1 à 2,1 : 1.

**8.** Procédé selon la revendication 1, caractérisé en ce que l'hydrogène est introduit en quantité correspondant à un rapport molaire de 0,5 : 1 à 10 : 1 avec l'aniline de formule II.

**9.** Procédé selon la revendication 8, caractérisé en ce que l'hydrogène est introduit en quantite correspondant à un rapport molaire de 0,8 : 1 à 1,2 : 1 avec l'aniline de formule II.

**10.** Procédé selon la revendication 1, caractérisé en ce que le catalyseur consiste en 0,5 à 6% de platine, 0,1 à 2% d'au moins un composé des éléments lithium, sodium, potassium, rubidium, caesium, béryllium, magnésium, calcium, strontium ou baryum et au moins un composé des éléments cuivre, germanium, étain, plomb, titane, niobium, rhénium, manganèse et ruthénium, avec un rapport atomique de 2,5 : 1 à 3,5 : 1 entre le platine et la somme de ces éléments.

**11.** Procédé selon la revendication 1, caractérisé en ce que le catalyseur consiste en 0,5 à 6% de platine, 0,1 à 2% d'au moins un composé des éléments lithium, sodium, potassium, rubidium, caesium, béryllium, magnésium, calcium, strontium ou baryum, et un composé du rhénium, avec un rapport atomique de 0,5 : 1 à 1,5 : 1 entre le platine et le rhénium.

**12.** Procédé selon la revendication 1, caractérisé en ce que $R_1$ représente un groupe alkyle en C1-C3 ou

l'hydrogène, $R_2$ représente un groupe alkyle en C1-C3 ou alcoxy en C1-C2, $R_3$ représente un groupe (alcoxy en C1-C3)-méthyle, $R_4$ représente un groupe méthyle ou l'hydrogène, $R_3$ et $R_4$ pouvant également former avec l'atome de carbone auquel ils sont reliés un groupe cyclohexyle, 2-méthylcyclohexyle ou 2,6-diméthylcyclohexyle.

**13.** Procédé selon la revendication 1, caractérisé en ce que $R_4$ représente un groupe alkyle en C1-C6, alcoxy en C1-C4, furfuryle ou tétrahydrofurfuryle.

**14.** Procédé selon la revendication 1, caractérisé en ce que $R_3$ et $R_4$ forment ensemble et avec l'atome de carbone auquel ils sont reliés un noyau pyranne ou tétrahydropyranne.

**15.** Procédé selon la revendication 1, caractérisé en ce que l'on fait réagir la 2-méthyl-6-éthyl-aniline avec le 1-méthoxypropanol-2 à un rapport molaire de 1 : 1 à 2,1 avec adjonction d'une mole d'hydrogène par mode de la 2-méthyl-6-éthyl-aniline en présence d'un catalyseur consistant en 3 à 5% de platine, 0,1 à 2% d'un composé du calcium, et un composé de l'étain-II en quantité correspondant à un rapport atomique platine/étain de 2,5 à 3,5, à 200° sous pression normale, pour conversion en la 2-méthyl-6-éthyl-N-(1-méthyl-2-méthoxy-éthyl)-aniline.

**16.** Procédé selon la revendication 1, caractérisé en ce que l'on fait réagir la 2,6-diéthylaniline avec le 2-propoxyéthanol à un rapport molaire de 1 : 1 à 2,1 avec adjonction d'une mode d'hydrogène par mode de 2,6-diéthylanidine en présence d'un catalyseur consistant en 3 à 5% de platine, 0,1 à 2% d'un composé du calcium et un composé de l'étain-II en quantité correspondant à un rapport atomique platine/étain de 2,5 à 3,5, à 190° et à pression normale, pour conversion en la 2,6-diéthyl-N-2-propoxyéthydaniline.

**17.** Catalyseur à base d'un gel de silice à une surface spécifique de 300 à 700 $m^2/g$, un volume de pores de 0,25 à 1,0 ml/g, un rayon de pore moyen de 1 à 8 nm, une densité apparente de 0,5 à 0,9 g/ml, une teneur en humidité de 1 à 5% et une teneur totale en fer, aluminium et titane inférieure à 750 ppm, ce gel de silice constituant le support, et contenant :

a) 0,5 à 6% de platine,

b) 0,1 à 2% d'au moins un composé d'un élément des groupes Ia et/ou IIa de la Classification Périodique, et

c) au moins un composé d'un élément des groupes Ib, IVa, IVb, Vb, VIIb et VIII, avec un rapport atomique de 1 : 1 à 6 : 1 entre le platine et la somme des éléments des groupes b) et c).

**18.** Catalyseur selon revendication 17, caractérisé en ce que le gel de silice a une surface spécifique de 400 à 600 $m^2/g$, un volume de pores de 0,35 à 0,75 ml/g, un rayon de pore moyen de 2 à 4 nm et une densité apparente de 0,65 à 0,75 g/ml, il contient de 1,5 a 2,5% d'humidité et sa teneur totale en fer, aluminium et titane ne dépasse pas 500 ppm.

**19.** Catalyseur à base de gel de silice qui sert de support, contenant :

a) 0,2 à 10% de platine,

b) 0,05 à 3% d'un composé d'un élément des groupes Ia et/ou IIa de la Classification Périodique, et

c) au moins un composé d'un élément des groupes Ib, IVa, IVb, Vb, VIIb et VIII, avec un rapport atomique de 1 : 1 à 6 : 1 entre le platine et la somme des éléments des groupes b) et c).

**20.** Catalyseur selon la revendication 19, consistant en 0,5 à 6% de platine, 0,1 à 2% d'au moins un composé des éléments lithium, sodium, potassium, rubidium, caesium, béryllium, magnésium, calcium, strontium ou baryum et au moins un composé des éléments cuivre, germanium, étain, plomb, titane, niobium, manganèse et ruthénium, avec un rapport atomique de 2,5 : 1 à 3,5 : 1 entre le platine et la somme de ces éléments.

**21.** Catalyseur selon la revendication 19, consistant en 0,5 à 6% de platine, 0,1 à 2% d'au moins un composé des éléments lithium, sodium, potassium, rubidium, caesium, béryllium, magnésium, calcium, strontium ou baryum, et un composé du rhénium, avec un rapport atomique de 0,5 : 1 à 1,5 : 1 entre le platine et le rhénium.

**22.** Catalyseur selon la revendication 19, consistant en 3 à 5% de platine, 0,1 à 2% d'un composé du

calcium, et un composé de l'étain-II, avec un rapport atomique de 2,5 : à 3,5 : 1 entre le platine et l'étain.